# EUROPÄISCHE PATENTSCHRIFT

(11) **EP 2 636 417 B1**
(45) Veröffentlichungstag und Bekanntmachung des Hinweises auf die Patenterteilung: **26.04.2017**
(21) Anmeldenummer: 12002332.0
(22) Anmeldetag: 05.03.2012
(51) Int. Cl.: A61M 1/00, A61F 13/02

(54) **Wundversorgungsanordnung und Abdeckeinrichtung dafür**
Wound treatment assembly and covering device for same
Agencement de pansement de plaies et dispositif de revêtement correspondant

(43) Veröffentlichungstag der Anmeldung: 11.09.2013
(73) Patentinhaber: Lohmann & Rauscher GmbH, 2525 Schönau/Triesting (AT)
(72) Erfinder: Grillitsch, Peter, 1130 Wien (AT); Kainz, Sonja, 1130 Wien (AT); Danei, Federico, 1180 Wien (AT); Steinlechner, Erik, Dr., 2500 Baden (AT)
(74) Vertreter: Leinweber & Zimmermann

(56) Entgegenhaltungen:
- EP-B1- 1 162 932
- WO-A1-2010/011148
- WO-A2-2010/008167
- US-A1- 2005 101 940
- US-A1- 2005 203 452
- US-A1- 2007 032 755
- US-A1- 2010 305 524
- US-A1- 2011 112 492
- US-A1- 2011 144 599
- US-A1- 2011 257 572

## Beschreibung

Die Erfindung betrifft eine Wundversorgungsanordnung nach dem Oberbegriff des Patentanspruchs 1 sowie eine Abdeckeinrichtung für eine solche Wundversorgungsanordnung.

Derartige Wundversorgungsanordnungen werden insbesondere im Rahmen der sogenannten Vakuumtherapie eingesetzt. Es hat sich gezeigt, daß im Besonderen die Heilung chronischer Wunden durch Anlegen von Unterdruck an diese Wunden gefördert werden kann. Dabei hat es sich weiter als vorteilhaft erwiesen, wenn die Wunde mit einem offenporigen Schaum oder Gaze als Füllmaterial abgedeckt bzw. gefüllt wird, die Wunde zur Erzeugung eines die Wunde und gegebenenfalls das Füllmaterial enthaltenden abgeschlossenen Wundraums abgedeckt wird und auf der der Wunde bzw. dem Füllmaterial abgewandten Seite der Abdeckeinrichtung ein Sauganschluß angebracht wird, über den der Wundraum mit einer zum Erzeugen von Unterdruck ausgelegten Saugeinrichtung verbunden werden kann. Bei anderen Anordnungen wird ein Flansch des Sauganschlusses von der Abdeckeinrichtung abgedeckt
oder in einer eine Öffnung der Abdeckung umlaufenden Tasche aufgenommen. Der Sauganschluß kann beispielsweise mit einem einerseits an einer beispielsweise in Form eines Rohrstutzens ausgeführten Verbindungseinrichtung des Sauganschlusses und andererseits mit einem an eine Saugeinrichtung anschließbaren Schlauch ausgestattet sein. Die Abdeckeinrichtung kann beispielsweise als folienartiges Material ausgeführt sein, welches an die der Wunde benachbarte Hautfläche luftdicht angelegt wird.

Wundversorgungsanordnungen, die im Rahmen der Vakuumtherapie einsetzbar sind, sind beispielsweise in der EP 0 620 720 B1 beschrieben.

In der DE 10 2009 019 646 A1 ist eine zur Verbesserung des Exsudat-Management zwischen dem Füllmaterial und dem Wundgrund einzulegende Kontaktschicht beschrieben, die einen Drainageraum zwischen dem Füllmaterial und dem Wundgrund bildet.

Im Rahmen der Vakuumtherapie einsetzbare Sauganschlüsse, welche über einen Schlauch mit einer Saugeinrichtung verbunden werden können, sind beispielsweise in der WO 03/073970 A1, der WO 2008/014358 A2 und der WO 2009/124548 A1 beschrieben. Ein als Saugkopf bezeichneter Sauganschluß mit zur Strömungsführung im Bereich der der Wunde zugewandten Begrenzungsfläche des Sauganschlusses dienenden Vorsprüngen ist in der EP 1 018 967 B1 beschrieben. Ferner ist ein Sauganschluß mit einer an das Füllmaterial anzulegenden Anlagefläche in Form einer scheibenartigen Schale in der EP 1 088 569 B1 angegeben. Bei einem in der WO 2010/008167 A2 beschriebenen Sauganschluß sind auf der dem Füllmaterial zugewandten Begrenzungsfläche durch Stege begrenzte Kanäle gebildet, durch die das Wundexsudat in Richtung auf eine Absaugöffnung geleitet werden soll.

In der WO 2010/011148 A1 ist eine im Rahmen der Vakuumtherapie einsetzbare Wundversorgungsanordnung beschrieben, die einen über eine Extremität des menschlichen Körpers ziehbaren undurchlässigen Schlauch sowie einen zwischen der Wunde und dem Schlauch anzuordnenden perforierten Körper aufweist. Mit dem perforieren Körper wird zwischen dem undurchlässigen Schlauch und dem Wundgrund ein Raum geschaffen, in dem über einen dichtend an den undurchlässigen Schlauch anlegbaren Schlauchanschluß ein Unterdruck erzeugt werden kann. In der EP 1 162 932 B1 ist eine Wundversorgungsanordnung mit einer Umhüllung aus einem Kunststoffmaterial und einem in der Umhüllung enthaltenen fluidabsorbierenden Material beschrieben. Die in dieser Schrift beschriebene Wundversorgungsanordnung ist zum Schutz von Wunden gedacht. Sie ist mangels Schlauchanschluß für den Einsatz in der Vakuumtherapie nicht geeignet. In der nicht vorveröffentlichten europäischen Patentanmeldung mit der Anmeldenummer 11001737.3 ist eine Wundversorgungsanordnung nach dem Oberbegriff des Patentanspruchs 1 beschrieben. Beim Einsatz herkömmlicher Wundversorgungsanordnungen werden in vielen Fällen eine übermäßige Austrocknung und manchmal auch eine Mazeration der Wunde während der Vakuumtherapie beobachtet.

Wundversorgungsanordnungen nach dem Oberbegriff des unabhängigen Patentanspruchs 1 sind bekannt aus der WO 2010/011148 A1 und der US 2005/203452 A1. Wundversorgungsanordnungen mit wasserdampfdurchlässigen Abdeckeinrichtungen sind bekannt aus der US 2011/144599 A1, US 2011/112492 A1, US 2007/032755 A1, US 2005/101940 A1, US 2011/257572 A1 und US 2010/305524 A1.

Angesichts dieser Probleme im Stand der Technik liegt der Erfindung die Aufgabe zugrunde, eine einfach anlegbare Wundversorgungsanordnung bereitzustellen, mit der unter Einsatz der Vakuumtherapie zuverlässig die Heilung von Wunden gefördert werden kann.

Erfindungsgemäß wird diese Aufgabe durch die im kennzeichnenden Teil des Patentanspruchs 1 angegebene Weiterbildung der bekannten Wundversorgungsanordnungen gelöst.

Im Rahmen der Erfindung wird von der bekannten Erkenntnis Gebrauch gemacht, daß die grundsätzlich an eine Wundabdeckung für die Vakuumtherapie zu stellenden Anforderungen, wonach ein luftdichter und wasserdichter Wundraum erzeugt werden soll, der zusätzlich auch noch keimdicht, biokompatibel und hautfreundlich sein soll, nicht übermäßig beeinträchtigt wird, wenn die Abdeckeinrichtung unter Inkaufnahme einer Beeinträchtigung der Dichtigkeit wasserdampfdurchlässig gestaltet wird. Durch die Wasserdampfdurchlässigkeit wird einerseits eine übermäßige Austrocknung der Wunde durch Transport von Feuchtigkeit aus der Umgebung in den Wundraum, andererseits aber auch eine Mazeration der Wunde durch Abtransport übermäßiger Feuchtigkeit durch die Abdeckung ermöglicht. Es läßt sich durch Einsatz einer wasserdampfdurchlässigen Abdeckeinrichtung ein heilungsförderndes Milieu im Wundbereich bzw. Wundraum einstellen. Darüber hinaus wird durch die Wasserdampfdurchlässigkeit der Wundabdeckung auch noch eine mögliche Beeinträchtigung der die Wunde umgebenden Haut, an der die Abdeckeinrichtung festzulegen ist, reduziert.

Die gewünschte Einstellung des Milieus im Wundraum kann besonders zuverlässig erfolgen, wenn die Wasserdampfdurchlässigkeit der Abdeckeinrichtung zumindest abschnittweise 300 g/m²/24 h oder mehr, insbesondere 500 g/m²/24 h oder mehr, besonders bevorzugt 750 g/m²/24 h beträgt.

Im Sinne der Vermeidung einer übermäßigen Austrocknung der Wunde beträgt die Wasserdampfdurchlässigkeit der Abdeckeinrichtung zweckmäßigerweise weniger als 10.000 g/m²/24 h, insbesondere weniger als 5.000 g/m²/24 h, besonders bevorzugt 3.000 g/m²/24 h oder weniger. Die Angaben zu der Wasserdampfdurchlässigkeit beziehen sich auf Messungen gemäß DIN EN ISO 13726-2.

Sofern sich die Wunde im Bereich eines Gelenks befindet, muß auch darauf geachtet werden, daß die Abdeckeinrichtung ausreichend verformbar ist. Das kann sichergestellt werden, wenn die Abdeckeinrichtung eine nachgiebige Abdeckfolie mit einer Dicke von 0,5 bis 200 µm, insbesondere 1 bis 100 µm aufweist. Im Sinne einer Beobachtung der Wunde ohne Entfernung der Wundversorgungsanordnung davon hat es sich als günstig erwiesen, wenn eine durchsichtige Abdeckfolie eingesetzt wird. Bei einer besonders bevorzugten Ausführungsform der Erfindung weist die Abdeckeinrichtung ein Polyurethanpolymer, insbesondere ein aromatisches Polyurethanpolymer auf.

Wie vorstehend im Zusammenhang mit bekannten Wundversorgungsanordnungen bereits erläutert, hat es sich als günstig erwiesen, wenn ein zum Füllen der Wunde zwischen dem Wundgrund und der Abdeckeinrichtung ausgelegtes Füllmaterial vorgesehen ist, wobei vorzugsweise auf der dem Füllmaterial zugewandten Seite des Sauganschlusses eine Drainageschicht zum Einleiten des aus dem Füllmaterial abzusaugenden Exsudats in mindestens eine Absaugöffnung des Sauganschlusses vorgesehen ist. Wenn ein solcher Sauganschluß vorgesehen wird, sind weder kanalbildende Vorsprünge an der dem Füllmaterial zugewandten Begrenzungsfläche des Sauganschlusses noch eine Erweiterung der Saugfläche in Form einer scheibenartigen Schale erforderlich. Vielmehr reicht es aus, wenn die dem Füllmaterial zugewandte Begrenzungsfläche des Absauganschlusses flanschartig eben ausgeführt ist und von der Saugöffnung durchsetzt wird, weil die Einleitung des Wundexsudats aus dem Füllmaterial nicht durch eine besondere Beschaffenheit des Anschlusses, sondern durch die zwischen dem Anschluß und dem Füllmaterial angeordnete Drainageschicht bewirkt wird. Wenn die an die Drainageschicht anzulegende Anlagefläche des Sauganschlusses eben, d.h. ohne Vorsprünge, sei es in Form von Kanäle begrenzenden Stegen, sei es in Form von ringförmigen Vorsprüngen, wie bei den scheibenartigen Schalen gemäß EP 1 088 569 B1, ausgeführt ist, wird das Eindrücken vorspringender Strukturen in die Drainageschicht verhindert und damit eine zuverlässige Funktion der Drainageschicht gewährleistet.

An dieser Stelle wird ergänzend auch noch darauf hingewiesen, daß der Einsatz wasserdampfdurchlässiger Abdeckeinrichtungen nicht nur zur Schaffung eines gewünschten Milieus im Wundraum, sondern auch zur Schonung der die Wunde umgebenden Haut zu nutzen ist, weil eine Beschädigung der Haut, wie sie bei undurchlässigen Abdeckeinrichtungen beobachtet wird, ausgeschlossen werden kann.

Die Abdeckeinrichtung einer erfindungsgemäßen Wundversorgungsanordnung soll an der die Wunde umgebenden Haut festlegbar sein. Dazu kann die Abdeckeinrichtung selbst mit einer haftenden Beschichtung ausgestattet sein. Das Anlegen erfindungsgemäßer Abdeckeinrichtungen wird jedoch vereinfacht, wenn der Abdeckeinrichtung in Form einer unbeschichteten, schlauchartigen Abdeckfolie eine separate Haftfolie, insbesondere haftende Polyurethanfolie zugeordnet ist, mit der die Wundabdeckung nach Anlegen an die Wunde an der die Wunde umgebenden Haut festgelegt werden kann. Die Haftfolie kann in Form eines Folienbandes vorliegen. Im Rahmen der Erfindung können als Haftfolie insbesondere unter dem Handelsnamen Suprasorb F angebotene und verkaufte Haftfolien der Anmelderin zum Einsatz kommen. In diesem Zusammenhang ist auch zu beachten, daß die Wasserdampfdurchlässigkeit der Abdeckeinrichtung durch eine haftende Beschichtung deutlich beeinträchtigt wird. Sie kann auf weniger als ein Drittel der unbeschichteten Abdeckeinrichtung zurückgehen.

Die erfindungsgemäße Wundversorgungsanordnung ist mit besonderem Vorteil zur Behandlung von Wunden an den Extremitäten, z.B. Fuß, Knöchel, Unterschenkel, Arm, Hand, geeignet. Dabei weist die Abdeckeinrichtung erfindungsgemäß einen wasserdampfdurchlässigen Folienschlauch aufweisen, in den diese Extremität einführbar ist. Die schlauchförmige Abdeckeinrichtung wird über die Extremität gezogen und bezüglich der Wunde so positioniert, daß die Wunde mit der Abdeckeinrichtung dichtend abgedeckt wird. Anschließend kann die Abdeckeinrichtung mit der Haftfolie an der der Wunde benachbarten Haut befestigt werden. Dazu wird die Haftfolie von einem Wickel abgezogen und derart um ein Ende des Schlauchs gewickelt, daß sie einerseits an dem Schlauch und andererseits an der Haut haftet.

Die Anlage der Abdeckeinrichtung kann vereinfacht werden, wenn der Folienschlauch an einem axialen Ende zur Bildung einer strumpfartigen Anordnung insbesondere luftdicht verschlossen ist. Das kann durch Verschweißen (Ultraschall, Hitze, RF) oder durch Verkleben (z.B. mit einem Polyurethan-Kleber, Schmelzkleber und/oder Klebeband) erfolgen. Die Abdeckeinrichtung muß dann nur noch auf einer Seite durch einen klebenden Folienverband abgedichtet sein.

Im Sinne eines einfachen Anlegens der Abdeckeinrichtung weist diese zweckmäßigerweise einen Gleitreibungskoeffizienten im Bereich zwischen 0,7 und 1,2, gemessen gemäß ASTM 1894-08, auf. Dabei kann ein ausreichender Halt der Abdeckeinrichtung erreicht werden, wenn der Haftreibungskoeffizient zwischen 0,8 und 1,5, besonders bevorzugt zwischen 1 und 1,25, gemessen gemäß ASTM 1894-08, liegt. In diesem Zusammenhang hat es sich weiter als zweckmäßig erwiesen, wenn die Reißdehnung der Abdeckeinrichtung mehr als 100 % beträgt.

Das Anlegen der Abdeckeinrichtung an die die Wunde aufweisende Extremität kann weiter vereinfacht werden, wenn der oft nur sehr dünnen Abdeckeinrichtung eine nach Anlegen an die Wunde davon lösbare Stützanordnung, insbesondere eine Stützfolie zugeordnet ist. Die der Abdeckeinrichtung gegebenenfalls zugeordnete Stützfolie kann aus transparentem Polyester oder aus einem mehrschichtigen Material (z.B. Polyesterkern, beidseitig polyethylenbeschichtet) hergestellt und auf der folienartigen Abdeckeinrichtung durch einen Laminierungsprozeß befestigt sein. Nach Einführung der von der Wunde affizierten Extremität in die Abdeckeinrichtung und vor der Abdichtung durch klebende Folienverbände muß die Stützfolie abgelöst werden, um einen erfolgreichen Therapieverlauf zu gewährleisten.

Der Sauganschluß einer erfindungsgemäßen Wundversorgungsanordnung weist eine im Gebrauch dem Wundraum zugewandte Absaugöffnung und eine vorzugsweise als Rohrstutzen ausgeführte Verbindungseinrichtung zum Herstellen einer Verbindung zwischen der Absaugöffnung und einem Absaugschlauch auf, wobei der Rohrstutzen auf der dem Wundraum abgewandten Seite des Sauganschlusses angeordnet ist. Dabei kann die Absaugöffnung einen flanschartigen Anlagebereich des Sauganschlusses durchsetzen, wobei der Anlagebereich einen die Absaugöffnung vorzugsweise umlaufenden und vorzugsweise mit einem Haftmittel ausgestatteten Befestigungsbereich aufweist, mit dem der Sauganschluß auf eine dem Wundraum abgewandte Begrenzungsfläche der Abdeckeinrichtung aufgeklebt werden kann. Alternativ kann der Anlagebereich auch zumindest teilweise von der Abdeckeinrichtung abgedeckt sein.

Der Folienschlauch einer erfindungsgemäßen Wundversorgungsanordnung kann zum Herstellen einer Verbindung zwischen dem an dem Rohrstutzen anzuschließenden Absaugschlauch und dem Wundraum eine vorbereitete Durchbrechung aufweisen. Das erschwert allerdings die positionsgerechte Anlage der Abdeckeinrichtung im Bereich der Wunde. Daher ist es im Rahmen der Erfindung besonders bevorzugt, wenn der Folienschlauch in Umfangsrichtung vollständig geschlossen ist und lediglich mindestens eine axiale Öffnung aufweist, durch die die Extremität in den Schlauch eingeführt werden kann, wobei die der Absaugöffnung des Sauganschlusses zugeordnete Durchbrechung in dem Folienschlauch erst nach Anlegen an die Wunde hergestellt wird, wobei der Folienschlauch dazu beispielsweise aufgeschnitten werden kann und die Durchbrechung im Gebrauch zweckmäßigerweise von dem Befestigungsbereich des Sauganschlusses umlaufen wird.

Nachstehend werden im Rahmen der Erfindung mit besonderem Vorteil einsetzbare Sauganschlüsse im einzelnen erläutert:
Im Sinne einer besonders einfachen Anwendung erfindungsgemäßer Wundversorgungsanordnungen hat es sich als zweckmäßig erwiesen, wenn die gegebenenfalls vorzusehende Drainageschicht an der Anlagefläche befestigt, insbesondere auf die Anlagefläche aufgeklebt, aufgeschweißt, an die Anlagefläche geklammert und/oder genäht ist. Die Gesamtanordnung, bestehend aus Sauganschluß und Drainageschicht, kann dann als Ganzes an der gewünschten Stelle auf dem Füllmaterial bzw. der Abdeckeinrichtung positioniert werden.

Wie im Zusammenhang mit erfindungsgemäß ausgeführten Abdeckeinrichtungen bereits erläutert, hat es sich gezeigt, daß bei der Vakuumtherapie ein gleichzeitig mit dem Erzeugen des Unterdrucks erfolgender kontinuierlicher Lufteintritt in den Wundbereich den Heilungsvorgang weiter verbessert. Daher weist eine Wundversorgungsanordnung gemäß einer besonders bevorzugten Ausführungsform der Erfindung einen Sauganschluß auf, welcher neben einer zum Absaugen des Exsudats verwendeten Absaugöffnung auch noch eine insbesondere in dem flanschartigen Anlagebereich des Sauganschlusses angeordnete und zur Belüftung der Wunde dienende Belüftungsöffnung aufweist. Ein durch die Belüftungsöffnung möglicher kontinuierlicher Lufteintritt in den Wundbereich verursacht einen kontrollierten und kontinuierlichen Druckabfall bei Verbinden einer Saugeinrichtung mit der Absaugöffnung. Dadurch kann im Besonderen in Verbindung mit dem Einsatz einer wasserdampfdurchlässigen Abdeckeinrichtung die Entfernung des Exsudats weiter verbessert werden. Die zum Absaugen des Exsudats verwendete Pumpe erzeugt dann einen höheren Durchfluß und eine verbesserte Saugwirkung. Sie tritt öfter in Aktion und saugt mehr ab.

Zur Vermeidung einer Kontamination der Wunde ist der Belüftungsöffnung zweckmäßigerweise ein antibakterielles Filter zugeordnet, welches in der Belüftungsöffnung angeordnet sein oder die Belüftungsöffnung abdecken kann. Das Filter ist zweckmäßigerweise hydrophob und weist zum Erhalt der gewünschten Filterwirkung eine Porengröße von 0,001, insbes. 0,005, bevorzugt 0,02, besonders bevorzugt 0,1 bis 5 Mikrometer auf. Bei einer Porengröße von weniger als 0,001 Mikrometer wird die gewünschte Belüftung beeinträchtigt. Bei einer Porengröße von mehr als 5 Mikrometer wird die antibakterielle Wirkung kaum noch erzielt. Das Material des Filters kann insbesondere Polytetrafluorethylen enthalten. Durch Wahl der Porengröße des Filters wird auch der Luftdurchfluß im Wundbereich beeinflußt, wobei eine Verkleinerung der Poren zu einer Verminderung des Lufteintritts führt.

Zusätzlich oder alternativ zu der Belüftungsöffnung kann die Wundversorgungsanordnung auch einen der Absaugöffnung zugewandten Multilumenschlauch aufweisen, der insbesondere drei Lumen enthalten kann, von denen nur einer zum Absaugen des Exsudats, ein anderer zur kontrollierten Luftzufuhr und ein dritter zur Druckmessung direkt an der Wunde benutzt wird. Durch Verwendung des Multilumenschlauchs kann die Saugwirkung verbessert werden, ohne daß der Sauganschluß bauliche Änderungen benötigt. Allerdings wird die Saugwirkung bei Verwendung einer zusätzlichen Belüftungsöffnung noch weiter verbessert. Eine weitere Verbesserung ist erreichbar, wenn der als Verbindungseinrichtung dienende Rohrstutzen eine dem Multilumenschlauch entsprechende Anzahl an Lumen aufweist.

Im Sinne einer optimalen Wundversorgung ohne Entfernen einer erfindungsgemäßen Wundversorgungsanordnung hat es sich als zweckmäßig erwiesen, wenn der Sauganschluß zusätzlich zu der Absaugöffnung auch noch eine zum Zuführen eines Wundversorgungsprodukts ausgelegte und vorzugsweise den Anlagebereich durchsetzende Zuführöffnung aufweist, der bei einer besonders bevorzugten Ausführungsform der Erfindung auf der der Anlagefläche abgewandten Seite des Sauganschlusses eine weitere Verbindungseinrichtung, wie etwa ein weiterer Rohrstutzen, zum Herstellen einer Verbindung zwischen der Zuführöffnung und einem Zuführschlauch zugeordnet sein kann. Über die Zuführöffnung kann bspw. eine Spüllösung ggf. mit Medikamenten, Desinfektionsmitteln und dgl. in den Wundbereich eingebracht werden. Die Zuführöffnung kann ebenso wie die Absaugöffnung von der Drainageschicht abgedeckt sein. Allerdings ist es im Rahmen der Erfindung besonders bevorzugt, wenn diese Öffnung nicht von der Drainageschicht abgedeckt ist, um das Diffundieren der Spüllösung in den Wundbereich zu verbessern.

Zur weiteren Verbesserung des Exsudatmanagements kann die erfindungsgemäße Wundversorgungsanordnung eine zwischen dem Wundgrund und dem Füllmaterial angeordnete, eine wundseitige Drainage bewirkende Kontaktschicht aufweisen.

Im Rahmen der Erfindung hat es sich als besonders vorteilhaft erwiesen, wenn die Drainageschicht und/oder die Kontaktschicht, entsprechend den Wundabdeckungen gemäß der DE 10 2009 019 646 A1, zwei etwa parallel zueinander verlaufende bahnförmige Elemente aufweist, zwischen denen ein Drainageraum gebildet ist, dessen Tiefe in einer sich etwa senkrecht zu den bahnförmigen Elementen erstreckenden Tiefenrichtung eine Kapillarwirkung auf in dem Drainageraum aufgenommene Exsudate gewährleistet. Die Tiefe des Drainageraums kann dazu 5 mm oder weniger und 0,5 mm oder mehr betragen. Dabei weist jedes der bahnförmigen Elemente zweckmäßigerweise eine den Durchtritt von Körperflüssigkeit in den Drainageraum ermöglichende Öffnung auf, wobei mindestens eine Öffnung durch einen sich ausgehend von einem der bahnförmigen Elemente in Richtung auf die gegenüberliegende innere Begrenzungsfläche des anderen bahnförmigen Elements erstreckenden und in den Drainageraum mündenden Kanal gebildet ist, dessen Kanalwand einstückig mit dem bahnförmigen Element ausgeführt, insbesondere durch Perforation des bahnförmigen Elements gebildet ist.

Im Sinne einer besonders ausgeprägten Kapillarwirkung hat es sich als günstig erwiesen, wenn sich die Querschnittsfläche des Kanals in einer sich senkrecht zur Tiefenrichtung erstreckenden Ebene, ausgehend von dem bahnförmigen Element, in Richtung auf die gegenüberliegende andere Begrenzungsfläche, insbesondere zum Erhalt einer den Eintritt von Körperflüssigkeit in den Drainageraum begünstigenden Kapillarwirkung, verringert. Dabei kann mindestens ein bahnförmiges Element eine Vielzahl von vorzugsweise rasterförmig angeordneten Öffnungen aufweisen, wobei der Abstand zwischen benachbarten Öffnungen 15 mm oder weniger, vorzugsweise 5 mm oder weniger, insbesondere 3 mm oder weniger, beträgt und die Mündungen der in einem bahnförmigen Element angeordneten Öffnungen in einer Projektion längs der Tiefenrichtung zwischen den Mündungen der in dem anderen bahnförmigen Element angeordneten Öffnungen angeordnet sind, wobei sich mindestens ein Kanal in Tiefenrichtung über 50 % oder mehr der gesamten Tiefe des Drainageraums erstreckt.

Die Kanalwand der die Öffnung bildenden Kanäle ist in einer sich parallel zur Tiefenrichtung erstreckenden Schnittebene zumindest abschnittweise bogenförmig ausgeführt und geht stetig in die Begrenzungsfläche des bahnförmigen Elements über. Weitere Merkmale erfindungsgemäß einsetzbarer Kontakt- und/oder Drainageschichten sind in der DE 10 2009 019 646 A1 angegeben.

Wenn die Drainageschicht die Anlagefläche des Sauganschlusses nur teilweise bedeckt, wobei vorzugsweise zumindest die Absaugöffnung und das Filter abgedeckt sind, und ein die Drainageschicht umlaufender Befestigungsbereich an der Anlagefläche des Sauganschlusses vorgesehen ist, kann der Sauganschluß mit diesem Befestigungsbereich auf die Abdeckeinrichtung aufgeklebt werden. Dazu kann der Befestigungsbereich des Sauganschlusses mit einem geeigneten Klebstoff versehen sein. Alternativ kann statt Klebstoff auch etwa ein doppelseitiges Klebeband verwendet werden. Der Klebstoff (z. B. Acrylat, Silikon, Polyurethan) kann dabei teilweise (z. B. in Ringen), porös oder vollflächig aufgebracht sein. Auch das Klebeband kann teilweise (z. B. in Ringen) oder vollflächig angebracht sein. Das doppelseitige Klebeband kann außerdem auf beiden Seiten mit dem selben Klebstoff beschichtet sein (z. B. Acrylat, Silikon, Polyurethan), oder die zwei Seiten können mit zwei verschiedenen Klebstoffen beschichtet sein (insbes. auf der oberen Seite, in Kontakt mit der Anlagefläche 12, mit Silikonkleber und auf der unteren Seite mit Acrylatkleber). Sowohl der Klebstoff als auch das Klebeband können mit einer lösbaren Schutzschicht versehen sein.

Wenn die Drainageschicht die Anlagefläche des Sauganschlusses vollständig bedeckt, kann die bspw. folienartig ausgeführte Abdeckeinrichtung auf einem der Anlagefläche abgewandten und die bspw. in Form eines Rohrstutzens ausgeführte Verbindungseinrichtung des Sauganschlusses umlaufenden Befestigungsbereich des Sauganschlusses aufgeklebt sein.

Ebenso wie bei bekannten Wundversorgungsanordnungen zum Einsatz in der Vakuumtherapie kann das Füllmaterial erfindungsgemäßer Wundversorgungsanordnungen einen offenporigen Schaum oder Gaze aufweisen.

Wie der vorstehenden Erläuterung erfindungsgemäßer Wundversorgungsanordnungen zu entnehmen ist, zeichnet sich eine erfindungsgemäße Abdeckeinrichtung zur Verwendung in einer erfindungsgemäßen Wundversorgungsanordnung im Wesentlichen dadurch aus, daß sie eine Wasserdampfdurchlässigkeit von vorzugsweise 300 g/m²/24 h oder mehr aufweist, wobei sie als Polyurethanfolie, insbesondere als Folienschlauch mit an die Abmessungen menschlicher Extremitäten angepaßten Abmessungen ausgeführt sein kann. Die Polyurethanfolie einer erfindungsgemäßen Abdeckeinrichtung ist vorzugsweise wasserdicht, biokompatibel und weist eine Dicke zwischen 0,5 und 200 µm, insbesondere zwischen 1 und 100 µm auf. Die Wasserdampfdurchlässigkeit beträgt zweckmäßigerweise weniger als 2000 g/m²/24 h, insbesondere weniger als 1500 g/m²/24 h. Die Reißdehnung einer erfindungsgemäßen Abdeckeinrichtung kann mehr als 100 % betragen, wobei der Haftreibungskoeffizient zwischen 0,8 und 1,5 und der Gleitreibungskoeffizient zweckmäßigerweise zwischen 0,7 und 1,2 liegt. Eine erfindungsgemäße Wundversorgungsanordnung kann wie folgt verwendet werden:
Eine auf einer Extremität positionierte Wunde wird nach einer üblichen Unterdrucktherapieverbandsanlage bzw. nach Anlegen bzw. Einlegen eines Füllmaterials an bzw. in die Wunde, in die schlauchförmige Abdeckfolie eingeschoben, so daß die Folie den gesamten Wundbereich abdeckt und die schlauchförmige Abdeckfolie über die Ränder der Wunde übersteht. Die Folie ist unter Berücksichtigung ihrer Nachgiebigkeit zweckmäßigerweise so dimensioniert, daß sie stramm bzw. lückenfrei an den Wundrändem anliegt. Die Länge der schlauchförmigen Folie kann durch Schneiden an die Wundgröße angepaßt werden. Die schlauchförmige Folie kann auch als Endlosschlauch angeliefert und vor Ort durch Schneiden konfektioniert werden. Die so durch das Füllmaterial und die Deckfolie abgedeckte Folie wird durch marktübliche klebende Folienverbände, beispielsweise unter dem Handelsnamen Suprasorb F der Lohmann & Rauscher GmbH, an seinen zwei axialen Enden dichtend mit der die Wunde umgebenden Haut verbunden. Dazu werden die Folienverbände so angelegt, daß sie einerseits an der Abdeckfolie und andererseits an der Haut haften. Durch den Einsatz wasserdampfdurchlässiger Abdeckeinrichtungen im Rahmen der Erfindung wird eine vollständige Abdichtung des Wundbereichs durch klebende Folienverbände vermieden. Das führt zur Förderung der Wundheilung. Die Abdeckfolie kann vor und während der Positionierung bezüglich der Wunde an einer Stützfolie angebracht sein. Die Stützfolie muß nach Einführen der von der Wunde affizierten Extremität und vor der Abdichtung durch klebende Folienverbände abgelöst werden, um einen erfolgreichen Therapieverlauf zu gewährleisten. Sofern der als Abdeckeinrichtung eingesetzte Folienschlauch an einem seiner axialen Enden bei Vorabbildung eines stumpfförmigen Gebildes verschlossen ist, z.B. durch Verschweißen oder durch Verkleben, wird der Folienschlauch nur an einem axialen Ende dichtend mit der die Wunde umgebenden Haut verbunden werden.

Nachstehend wird die Erfindung unter Bezugnahme auf die Zeichnung, auf die hinsichtlich aller erfindungswesentlichen und in der Beschreibung nicht näher herausgestellten Einzelheiten ausdrücklich verwiesen wird, erläutert. In der Zeichnung zeigt:
- **Fig. 1**: einen Sauganschluß einer erfindungsgemäßen Wundversorgungsanordnung gemäß einer ersten Ausführungsform,
- **Fig. 2**: einen Sauganschluß einer erfindungsgemäßen Wundversorgungsanordnung gemäß einer zweiten Ausführungsform,
- **Fig. 3**: einen Sauganschluß einer erfindungsgemäßen Wundversorgungsanordnung gemäß einer dritten Ausführungsform,
- **Fig. 4**: eine mit dem Sauganschluß gemäß Fig. 3 ausgeführte Wundversorgungsanordnung,
- **Fig. 5**: einen Sauganschluß einer erfindungsgemäßen Wundversorgungsanordnung gemäß einer vierten Ausführungsform,
- **Fig. 6**: eine Wundversorgungsanordnung mit einem Sauganschluß gemäß Figur 5,
- **Fig. 7**: einen Sauganschluß einer erfindungsgemäßen Wundversorgungsanordnung gemäß einer fünften Ausführungsform,
- **Fig. 8**: eine Wundversorgungsanordnung mit einem Sauganschluß gemäß Fig. 7, und
- **Fig. 9**: einen Sauganschluß einer erfindungsgemäßen Wundversorgungsanordnung gemäß einer sechsten Ausführungsform.
- **Fig. 10**: Ausführungsformen erfindungsgemäßer Abdeckeinrichtungen für erfindungsgemäße Wundversorgungsanordnungen und
- **Fig. 11**: Anwendungsbeispiele erfindungsgemäßer Wundversorgungsanordnungen.

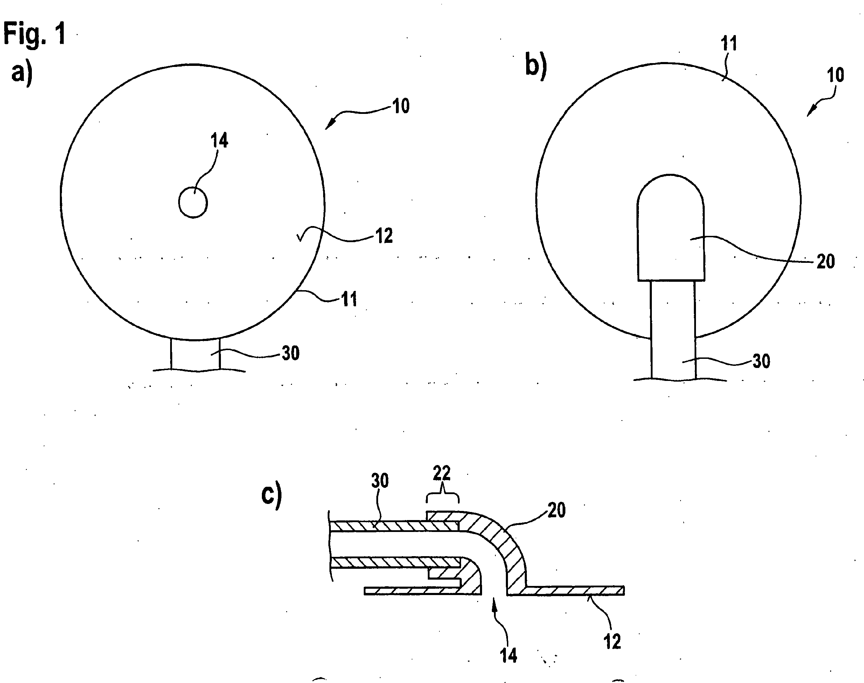

In Fig. 1a) ist eine Ansicht eines Sauganschlusses 10 einer erfindungsgemäßen Wundversorgungsanordnung von unten, in Fig. 1b) eine Ansicht des Sauganschlusses 10 gemäß Fig. 1a) von oben und in Fig. 1c) eine Schnittdarstellung eines erfindungsgemäßen Sauganschlusses 10 gezeigt.

Der in Fig. 1 dargestellte Sauganschluß 10 weist einen flanschartigen Anlagebereich 11 mit einer kreisscheibenförmigen, ebenen Anlagefläche 12 ohne Vorsprünge auf. Die Anlagefläche 12 ist von einer Absaugöffnung 14 durchsetzt. Dabei ist die ebenfalls kreisförmige Absaugöffnung 14 im Zentrum der Anlagefläche 12 angeordnet. Die Absaugöffnung 14 mündet in eine Verbindungseinrichtung 20, welche nach Art eines Rohrstutzens ausgeführt ist und eine Umlenkung der senkrecht zur Anlagefläche 12 ausgerichteten Exsudatströmung um 90° bewirkt, so daß die Strömung nach Umlenkung etwa parallel zur Anlagefläche 12 ausgerichtet ist. Der Rohrstutzen 20 weist an seinem der Absaugöffnung 14 abgewandten Ende einen Anschlußbereich 22 mit erweitertem Innendurchmesser auf, in den ein Absaugschlauch 30 luftdicht einführbar ist. Durch die Umlenkung der Absaugströmung mit Hilfe des Rohrstutzens 20 wird eine Ausrichtung des Absaugschlauchs 30 in einer parallel zur Anlagefläche 12 verlaufenden Richtung ermöglicht. Dadurch wird eine störungsarme Anlage des Sauganschlusses 10 und des damit verbundenen Absaugschlauchs 30 an dem Körper des Patienten ermöglicht.

Bei der in Fig. 1c) dargestellten Ausführungsform ist der Absaugschlauch 30 so dimensioniert, daß seine innere Begrenzungsfläche mit einem an den Anschlußbereich 22 angrenzenden Innenflächenbereich des Rohrstutzens 20 fluchtet, um so den Strömungswiderstand für das Wundexsudat zu minimieren.

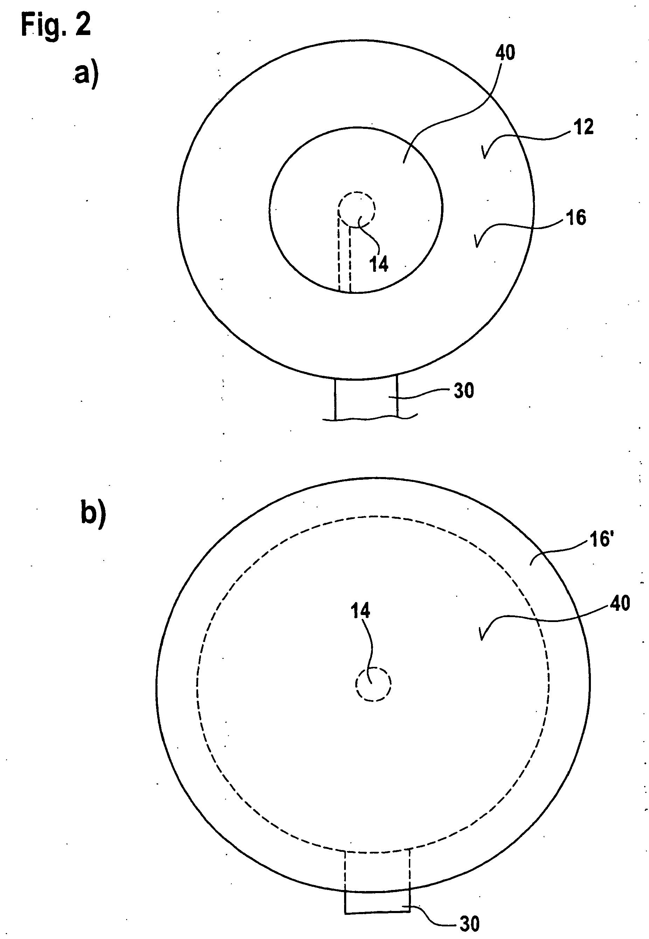

Bei der Ausführungsform gemäß Fig. 2a) ist ein die Absaugöffnung 14 enthaltender zentraler Bereich der Anlagefläche 12 von einer zum Einleiten von Wundexsudat in die Absaugöffnung 14 ausgelegten Drainageschicht 40 abgedeckt. Die Drainageschicht 40 ist auf die Anlagefläche 12 aufgeklebt und wird von einem Befestigungsbereich 16 der Anlagefläche 12 ringförmig umlaufen.

Bei der Ausführungsform gemäß Fig. 2b) ist die Anlagefläche 12 vollständig von der Drainageschicht 40 abgedeckt. Auf der der Anlagefläche 12 abgewandten Begrenzungsfläche des Sauganschlusses 10 ist ein den Rohrstutzen 20 umlaufender Befestigungsbereich 16' vorgesehen, welcher ebenso wie der Befestigungsbereich 16 mit einer Klebeschicht versehen sein kann, die vor Benutzung des Sauganschlusses 10 in einer erfindungsgemäßen Wundversorgungsanordnung von einer lösbaren Schutzschicht abgedeckt sein kann.

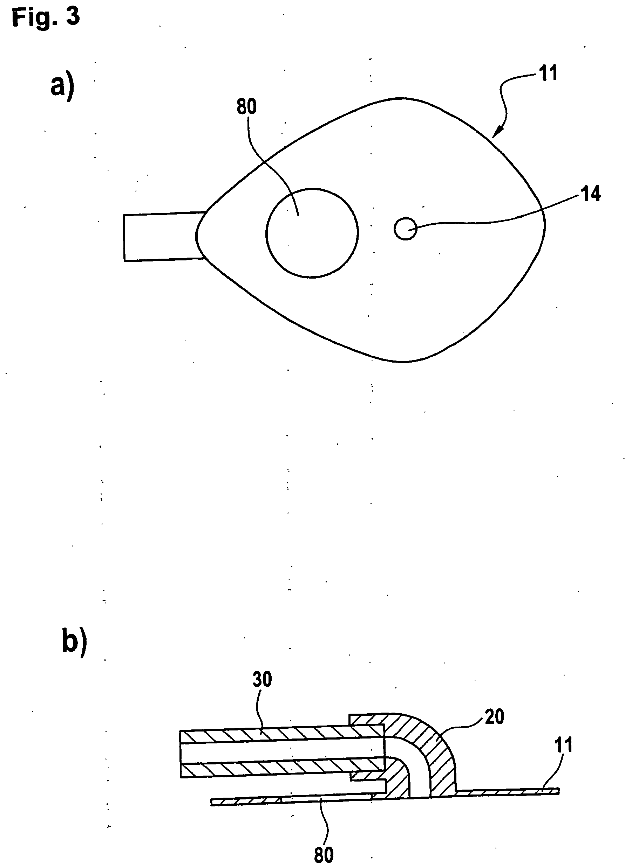

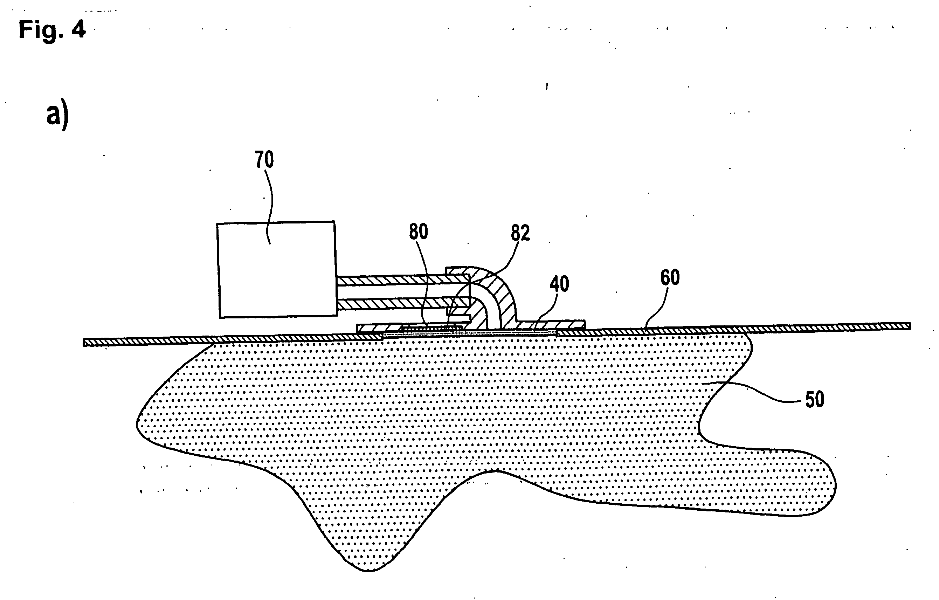

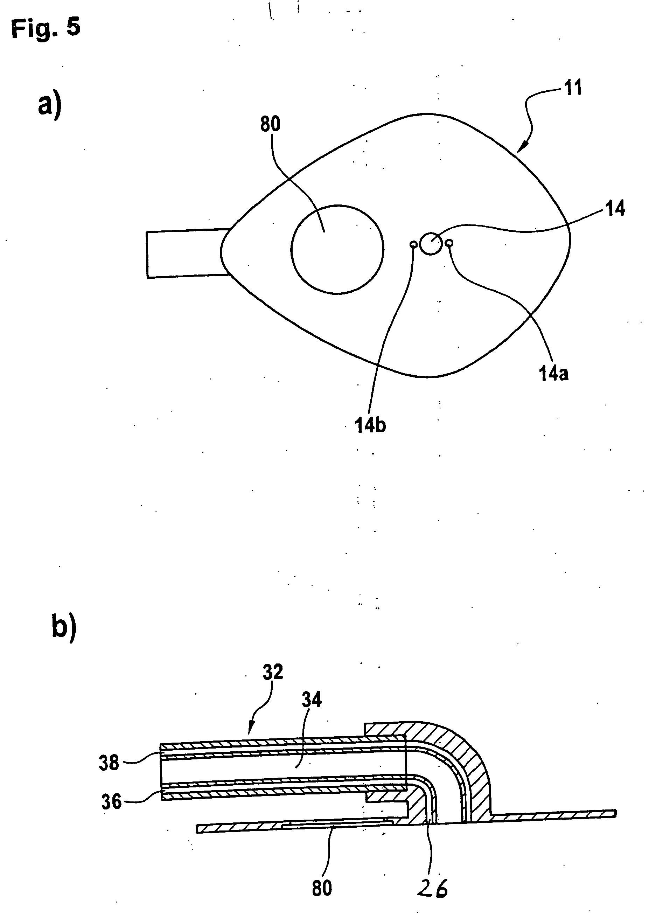

Der Sauganschluß gemäß Fig. 3 unterscheidet sich im wesentlichen dadurch von dem Sauganschluß gemäß den Fig. 1 und 2, daß der Anlagebereich 11 zusätzlich zu einer Ansaugöffnung 14 auch noch eine Belüftungsöffnung 80 aufweist, durch die die Wunde kontrolliert belüftet werden kann, um so die Strömungsverhältnisse im Wundbereich im Sinne einer Verbesserung der Exsudatabführung zu verbessern. Die Belüftungsöffnung 80 weist einen größeren Durchmesser auf als die Absaugöffnung 14. Der die Belüftungsöffnung 80 in dem Anlagebereich 11 umlaufende Rand kann einen Absatz aufweisen, der gemäß Fig. 4 und 5 als Auflagefläche für ein antibakterielles Filter 82 eingesetzt werden kann. Dadurch wird der Strömungswiderstand im Bereich der Belüftungsöffnung 80 erhöht, was allerdings durch Einstellung des Durchmessers der Belüftungsöffnung 80 wieder ausgeglichen werden kann.

Im Rahmen der Erfindung kann der Strömungswiderstand des Filters 82 ausgenutzt werden, um die Luftströmung zu steuern. Dabei wird der Strömungswiderstand durch Verkleinerung der Filterporen vergrößert. Diese Steuerung begünstigt die Erzeugung des Unterdrucks bei gleichzeitiger Belüftung. Sofern die Belüftungsöffnung 80 zu groß gewählt wird, kann der Unterdruck ohne Einsatz eines Filters nicht erzeugt werden. Die Belüftungsöffnung 80 kann auch über der wasserdampfdurchlässigen Abdeckfolie 60 angeordnet sein (in der Draufsicht). Wesentlich ist, daß der Wunde Luft zugeführt wird. Dazu kann es zweckmäßig sein, etwas versetzt ein Loch in der Abdeckfolie 60 vorzusehen, damit die Luft ungehindert zur Belüftungsöffnung 80 strömen kann.

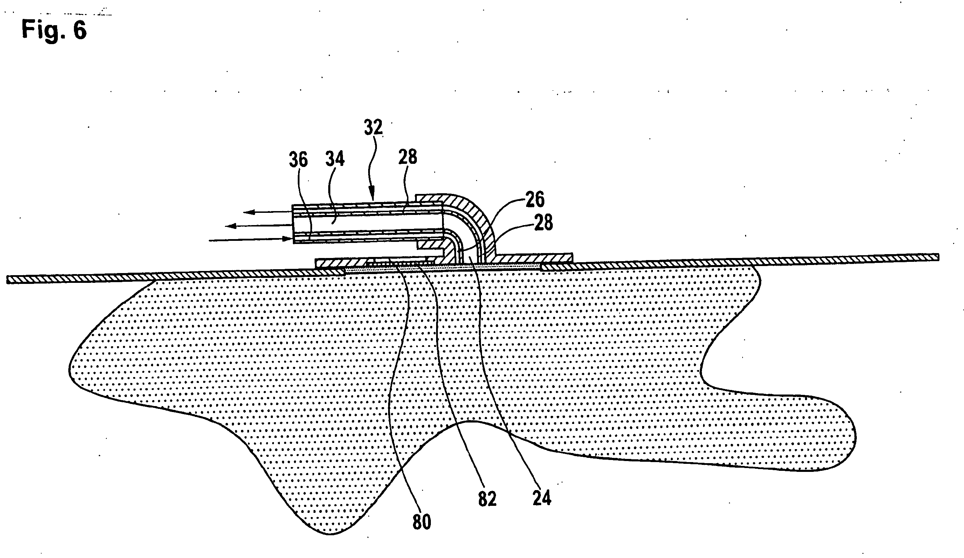

Das antibakterielle Filter 82 ist bei der in der Zeichnung dargestellten Ausführungsform aus Polytetrafluorethylen gebildet und weist eine Porengröße im Bereich von 0,001, insbes. 0,005, bevorzugt 0,02, besonders bevorzugt 0,1 bis 5 Mikrometern auf. Es wird bei der Wundversorgungsanordnung gemäß Fig. 6a und 6b von der Drainageschicht 40 abgedeckt. Wie anhand der Fig. 4 erläutert, kann der Sauganschluß gemäß Fig. 3 ebenso wie der Sauganschluß gemäß Fig. 1 so angebracht werden, daß er auf einer Abdeckfolie 60 aufgeklebt ist.

Die Ausführungsform gemäß Fig. 5 unterscheidet sich im wesentlichen dadurch von der Ausführungsform gemäß Fig. 3, daß der Absaugschlauch als Dreilumenschlauch 32 verwirklicht ist, wobei wie in Fig. 6 schematisch angedeutet ist, das mittlere und größte Lumen 34 zum Erzeugen des Unterdrucks im Wundbereich benutzt wird, ein kleines Lumen 36 zum Belüften der Wunde benutzt werden kann und ein weiteres kleineres Lumen 38 zum Messen des Drucks im Wundbereich gedacht ist. Der Anschlußbereich 22 weist ebenso den Lumen 32, 34 und 36 entsprechende Lumen 24, 26 und 28 auf.

Die Ausführungsform gemäß den Fig. 5 und 6 weist neben dem Belüftungslumen 26 auch noch eine Belüftungsöffnung 80 auf, um das Exsudat-Management weiter verbessern zu können.

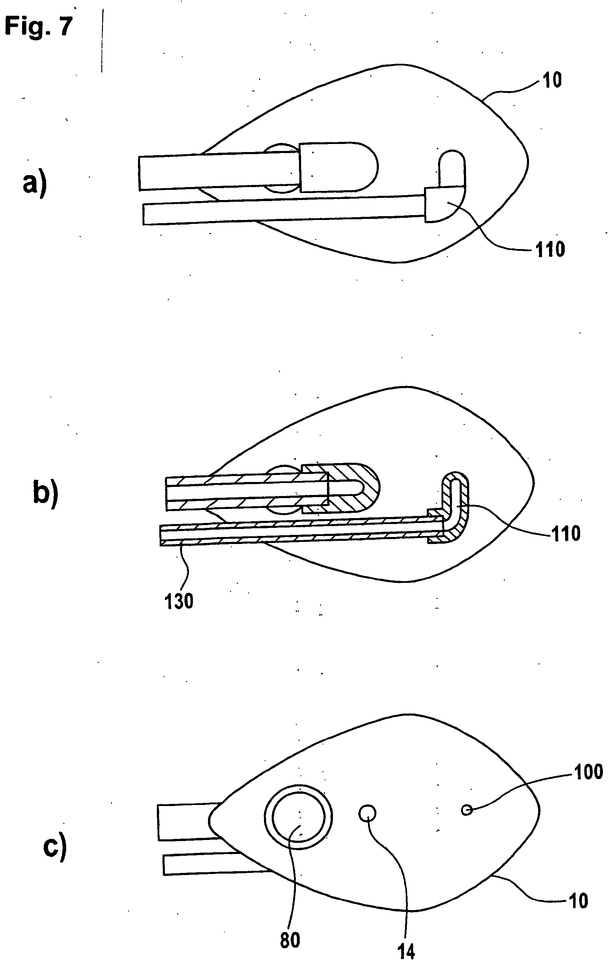

Die Ausführungsform gemäß Fig. 7 unterscheidet sich im wesentlichen dadurch von der Ausführungsform gemäß Fig. 3, daß zusätzlich zu der Absaugöffnung 14 und der Belüftungsöffnung 80 auch noch eine Zuführöffnung 100 in den Anlagebereich 11 des Sauganschlusses 10 vorgesehen ist, der ein weiterer Rohrstutzen 110 zum Verbinden der Zuführöffnung 100 mit einem weiteren Schlauch 130 zugeordnet ist. Über den Schlauch 130, den weiteren Rohrstutzen 110 und die Zuführöffnung 100 können Wundversorgungsmittel wie etwa eine ggf. auch mit Medikamenten, Desinfektionsmitteln oder dgl. versetzte Spüllösung, in den Wundbereich eingebracht werden. Die Ausführungsform gemäß Fig. 7 kann auch ohne Verwendung einer separaten Belüftungsöffnung 80 benutzt werden, weil die Zuführöffnung 100 auch zum Belüften eingesetzt werden kann. Im Sinne eines guten Exsudat-Managements hat es sich allerdings als besonders zweckmäßig erwiesen, wenn sowohl eine Belüftungsöffnung 80 als auch eine Zuführöffnung 100 neben der Absaugöffnung 14 in dem Anlagebereich 11 des Sauganschlusses 10 vorgesehen sind.

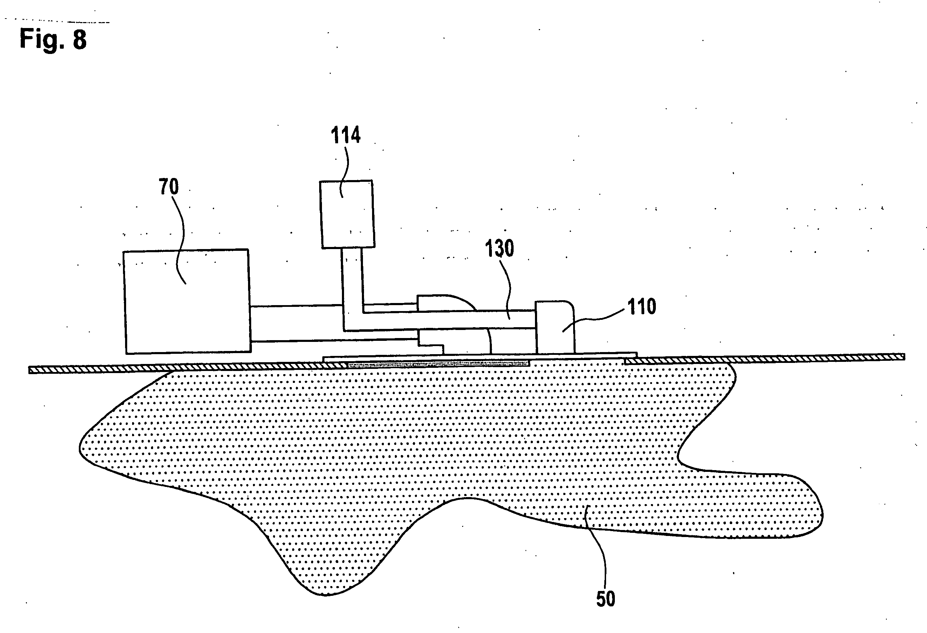

Wie in Fig. 8 erkennbar ist, kann die Spüllösung über den weiteren Schlauch 130 und den weiteren Rohrstutzen 110 mit Hilfe eines entsprechenden Dosierelements 114 in den Bereich der Wunde eingebracht werden. Dabei hat es sich als besonders zweckmäßig erwiesen, wenn die Zuführöffnung 100 nicht von der Drainageschicht 40 abgedeckt wird, um so das Absaugen von Wundexsudat ohne Beeinflussung durch die zugeführte Spüllösung zu gewährleisten und um zu verhindern, daß die zugeführte Spüllösung sofort wieder abgesaugt wird.

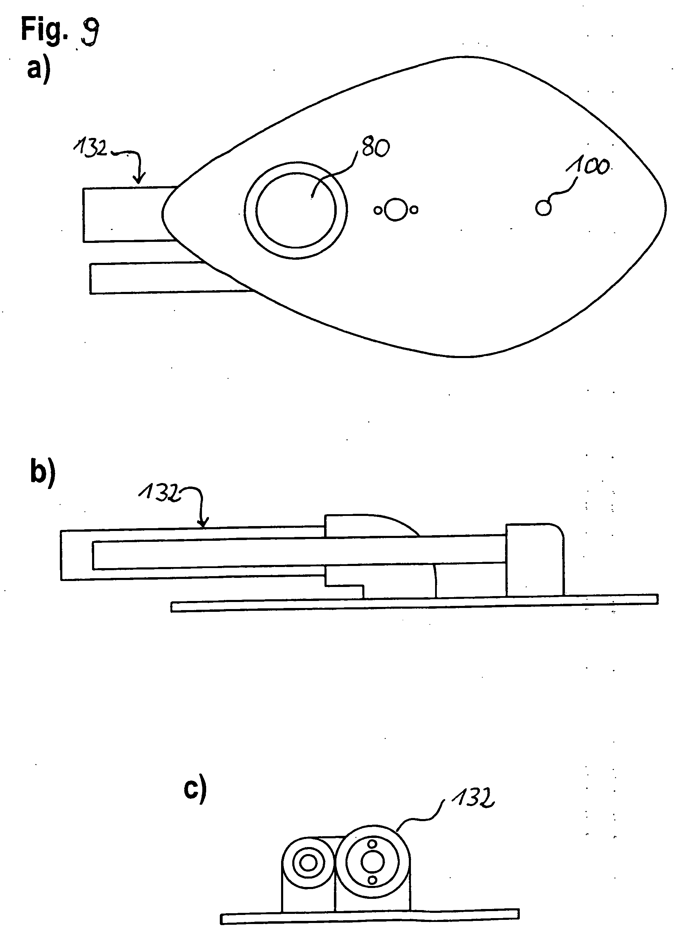

Die Ausführungsform gemäß Fig. 9 unterscheidet sich dadurch von der anhand der Fig. 7 erläuterten Ausführungsform, daß ein Dreilumenschlauch 132 zum Einsatz kommt, dessen Ausführung und Funktion der Ausführung gemäß Fig. 5 vergleichbar ist. Hierzu weist die Ausführung gemäß Fig. 9 einen Dreilumenschlauch zum Absaugen von Wundexsudat, Belüften des Wundbereichs und zur Druckmessung eine Belüftungsöffnung 80 sowie eine Zuführöffnung 100 auf. Dadurch kann ein optimales Wundmanagement gewährleistet werden.

Die anhand der Fig. 3 bis 9 erläuterten Ausführungsformen weisen anders als die anhand der in den Fig. 1 und 2 erläuterten Ausführungsformen einen Anlagebereich mit einer von einer Kreisform abweichender Form, nämlich etwa rautenförmiger Form auf. Die Ecken der Rauten sind abgerundet. Durch die Rautenform wird eine Längsachse bereitgestellt, welche die Anordnung von Absaugöffnung, Belüftungsöffnung und Zuführöffnung auf einer Linie erlaubt.

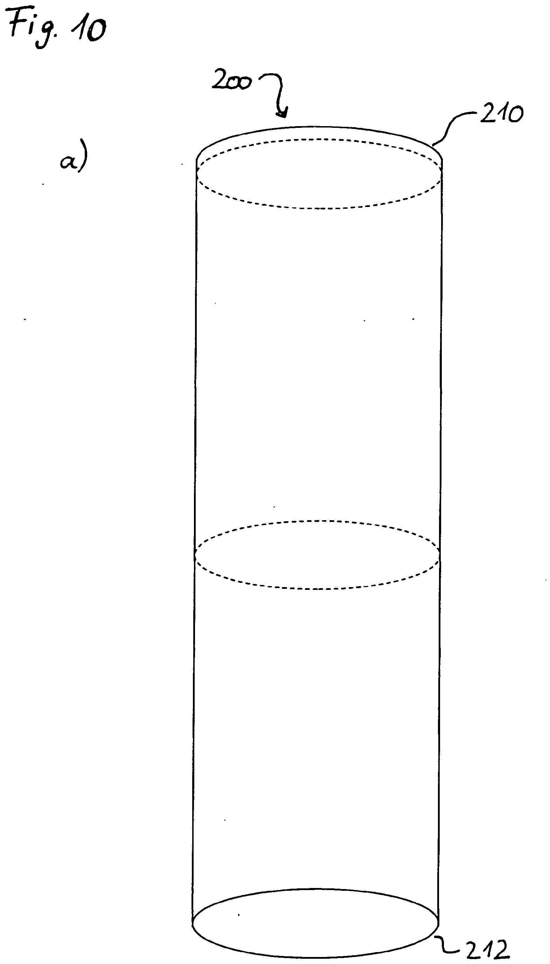

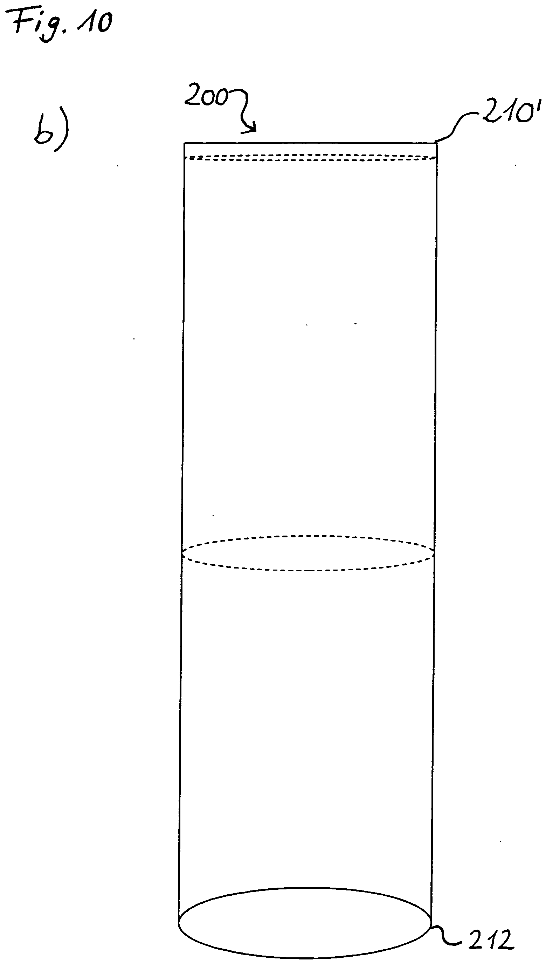

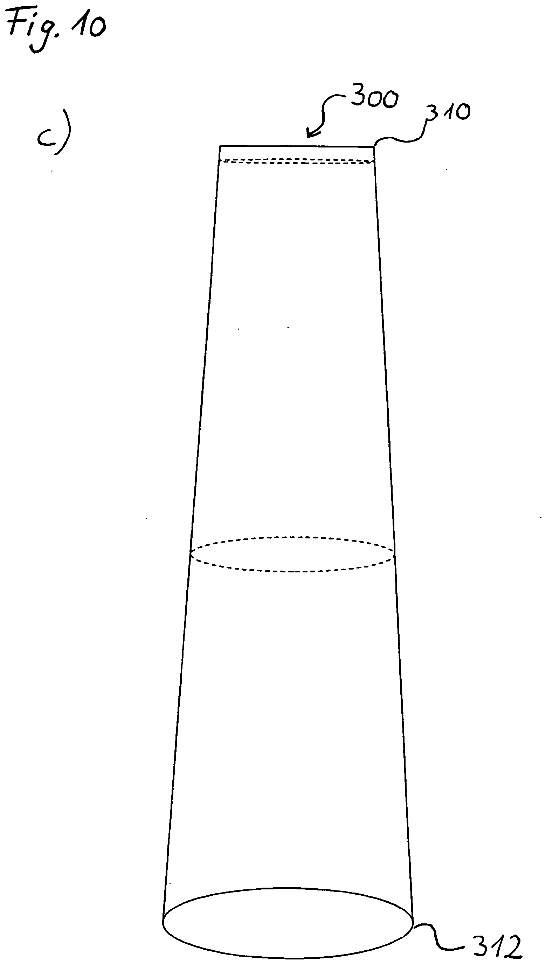

Die in Fig. 10 a) dargestellte Abdeckeinrichtung ist in Form eines Folienschlauchs 200 ausgeführt, welcher eine kreiszylindermantelförmige Gestalt annehmen kann. Die beiden Enden 210 und 212 des in Fig. 10 a) dargestellten Folienschlauchs sind offen ausgeführt, so daß der Schlauch insgesamt über eine Extremität gezogen werden kann. Die erfindungsgemäße Abdeckeinrichtung gemäß Fig. 10 b) unterscheidet sich im Wesentlichen dadurch von der Abdeckeinrichtung gemäß Fig. 10 a), daß das obere Ende 210' des hohlen Schlauchs 200 geschlossen ist.

Bei der Ausführungsform gemäß Fig. 10 c) ist die als Folienschlauch ausgeführte Abdeckeinrichtung in Kegelstumpfmantelform, also konisch ausbreitbar. Dabei kann ein Ende, etwa das obere Ende 310, geschlossen sein, während das andere, wie etwa das untere Ende 312, offen ausgebildet sein kann. Besonders bevorzugt ist das Ende mit dem geringeren Querschnitt geschlossen. Der Folienschlauch kann dann etwa nach Art eines Strumpfes über einen Fuß oder ggf. nach Art eines Handschuhs über eine Hand gestülpt werden.

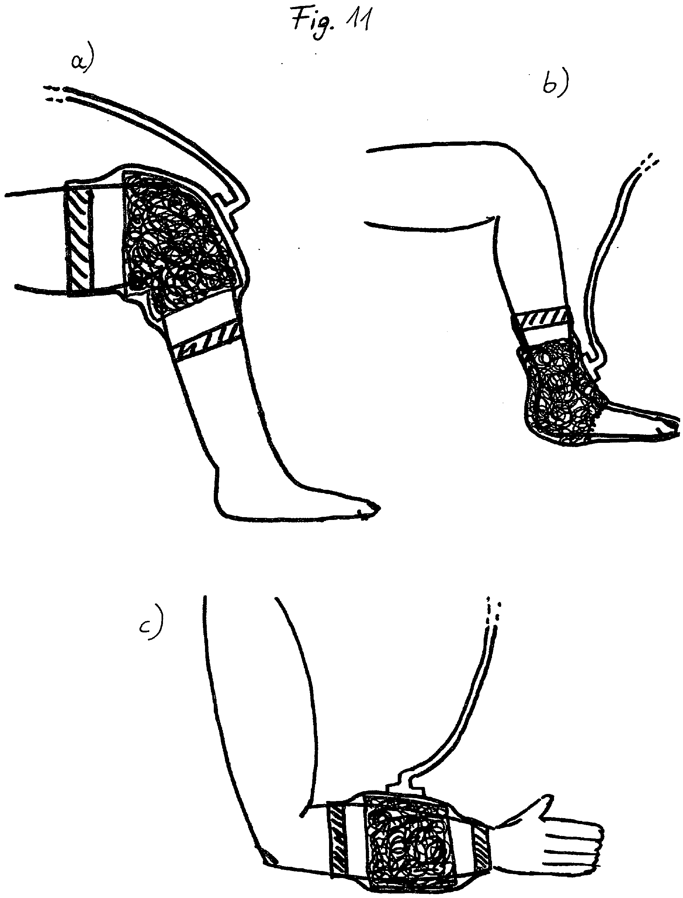

In Fig. 11 sind verschiedene Anwendungsbeispiele erfindungsgemäßer Wundversorgungsanordnungen dargestellt. Dabei zeigt Fig. 11a) die Anwendung im Bereich des Kniegelenks. Erkennbar ist, daß die Abdeckeinrichtung nach Art der in Fig. 10 c) oder 10 a) dargestellten Abdeckeinrichtung beidseitig offen schlauchförmig ausgeführt ist und an den einander entgegengesetzten Enden mit Hilfe von Haftmitteln an der umgebenden Haut befestigt ist.

Im Anwendungsbeispiel gemäß Fig. 11b) ist die Wundversorgungsanordnung an einem Knöchel angelegt. Die Abdeckeinrichtung ist bei diesem Beispiel nach Art eines geschlossenen Folienschlauchs ausgeführt, welcher im Bereich der Wade des Benutzers an der umgebenden Haut befestigt ist.

Schließlich ist die Wundversorgungsanordnung bei dem in Fig. 11c) dargestellten Anwendungsbeispiel am Unterarm eines Patienten angelegt. Bei diesem Beispiel ist die Abdeckeinrichtung als beidseitig offener Folienschlauch ausgeführt, wobei der Folienschlauch entsprechend der Darstellung in Fig. 10 c) konisch ausgeführt ist und an den beiden entgegengesetzten Rändern haftend an der umgebenden Haut befestigt ist.

## Patentansprüche

1. Wundversorgungsanordnung mit einer an der eine Wunde umgebenden Haut festlegbaren und zum Herstellen eines die Wunde enthaltenden abgeschlossenen Wundraums dienenden Abdeckeinrichtung und einem Sauganschluß (10) über den ein Unterdruck im Wundraum erzeugt werden kann, wobei die Abdeckeinrichtung einen Folienschlauch (200) aufweist, in den eine menschliche Extremität einführbar ist, **dadurch gekennzeichnet, daß** die Abdeckeinrichtung zumindest abschnittweise wasserdampfdurchlässig ist und die Wasserdampfdurchlässigkeit der Abdeckeinrichtung zumindest abschnittweise mehr als 300 g/m²/24 h beträgt.

2. Wundversorgungsanordnung nach Anspruch 1, **dadurch gekennzeichnet, daß** die Wasserdampfdurchlässigkeit der Abdeckeinrichtung weniger als 10.000 g/m²/24 h, insbesondere weniger als 5.000 g/m²/24 h, besonders bevorzugt 3.000 g/m²/24 h oder weniger beträgt.

3. Wundversorgungsanordnung nach einem der vorhergehenden Ansprüche, **dadurch gekennzeichnet, daß** die Abdeckeinrichtung eine vorzugsweise durchsichtige Abdeckfolie mit einer Dicke von 0,5 bis 200 µm, insbesondere 1 bis 100 µm aufweist.

4. Wundversorgungsanordnung nach einem der vorhergehenden Ansprüche, **dadurch gekennzeichnet, daß** die Reißdehnung der Abdeckeinrichtung 100 % oder mehr beträgt.

5. Wundversorgungsanordnung nach einem der vorhergehenden Ansprüche, **dadurch gekennzeichnet, daß** der Haftreibungskoeffizient der Abdeckeinrichtung (ASTM 1894-08) zwischen 0,8 und 1,5 beträgt.

6. Wundversorgungsanordnung nach einem der vorhergehenden Ansprüche, **dadurch gekennzeichnet, daß** der Gleitreibungskoeffizient (ASTM 1894-08) der Abdeckeinrichtung zwischen 0,7 und 1,2 beträgt.

7. Wundversorgungsanordnung nach einem der vorhergehenden Ansprüche, **dadurch gekennzeichnet, daß** die Abdeckeinrichtung ein Polyurethanpolymer, insbesondere ein aromatisches Polyurethanpolymer aufweist.

8. Wundversorgungsanordnung nach einem der vorhergehenden Ansprüche, **gekennzeichnet durch** ein zum Füllen des Wundraums zwischen dem Wundgrund und der Abdeckeinrichtung ausgelegtes Füllmaterial, wobei vorzugsweise auf der dem Füllmaterial zugewandten Seite des Sauganschlusses eine Drainageschicht zum Einleiten des aus dem Füllmaterial abzusaugenden Exsudats in mindestens eine Absaugöffnung des Sauganschlusses vorgesehen ist.

9. Wundversorgungsanordnung nach einem der vorhergehenden Ansprüche, **dadurch gekennzeichnet, daß** der Abdeckeinrichtung ein haftendes Befestigungsmittel zur haftenden Befestigung der Abdeckeinrichtung an der die Wunde umgebenden Haut zugeordnet ist.

10. Wundversorgungsanordnung nach Anspruch 9, **dadurch gekennzeichnet, daß** das Befestigungsmittel eine separate Haftfolie, insbesondere Polyurethanfolie aufweist, mit der die Wundabdeckung nach Anlegen an die Wunde bezüglich der die Wunde umgebenden Haut festlegbar ist.

11. Wundversorgungsanordnung nach einem der vorhergehenden Ansprüche, **dadurch gekennzeichnet, daß** der Folienschlauch an einem axialen Ende zur Bildung einer stumpfartigen Anordnung verschlossen, insbesondere luftdicht verschlossen ist.

12. Wundversorgungsanordnung nach einem der vorhergehenden Ansprüche, **dadurch gekennzeichnet, daß** der Abdeckeinrichtung eine nach Anlegen an die Wunde davon lösbare Stützanordnung, insbesondere Stützfolie zugeordnet ist.

13. Wundversorgungsanordnung nach einem der vorangehenden Ansprüche, **dadurch gekennzeichnet, daß** der Sauganschluß eine im Gebrauch dem Wundraum zugewandte Absaugöffnung und eine vorzugsweise als Rohrstutzen ausgeführte Verbindungseinrichtung zum Herstellen einer Verbindung zwischen der Absaugöffnung und einem Absaugschlauch aufweist.

14. Wundversorgungsanordnung nach Anspruch 13, **dadurch gekennzeichnet, daß** die Absaugöffnung einen flanschartigen Anlagebereich des Sauganschlusses durchsetzt und der Anlagebereich einen die Absaugöffnung vorzugsweise umlaufenden und vorzugsweise mit einem Haftmittel ausgestatteten Befestigungsbereich aufweist, mit dem der Sauganschluß auf eine dem Wundraum abgewandte Begrenzungsfläche der Abdeckeinrichtung aufklebbar ist.

15. Wundversorgungsanordnung nach einem der vorhergehenden Ansprüche, **dadurch gekennzeichnet, daß** der Folienschlauch in Umfangsrichtung vollständig geschlossen ist und zum Erhalt einer der Absaugöffnung zugeordneten Durchbrechung vorzugsweise nach Anlegen an die Wunde aufgeschnitten wird, wobei die Durchbrechung im Gebrauch vorzugsweise von dem Befestigungsbereich des Sauganschlusses umlaufen wird.

## Claims

1. A wound care arrangement having a covering unit that can be attached to the skin surrounding a wound and used to produce an enclosed wound space comprising the wound, and a suction connection (10) across which a negative pressure can be generated in the wound space, the covering unit comprising a film tube (200) into which a human extremity can be introduced, **characterised in that** the covering unit is water vapour permeable, at least in sections, and the water vapour permeability of the covering unit is, at least in sections, more than 300 g/m²/24 hrs.

2. The wound care arrangement according to Claim 1, **characterised in that** the water vapour permeability of the covering unit is less than 10,000 g/m²/24 hrs, in particular less than 5,000 g/m²/24 hrs, particularly preferably 3,000 g/m²/24 hrs or less.

3. The wound care arrangement according to any of the preceding claims, **characterised in that** the covering unit comprises a preferably transparent cover film that has a thickness of 0.5 to 200 µm, in particular 1 to 100 µm.

4. The wound care arrangement according to any of the preceding claims, **characterised in that** the elongation at rupture of the covering unit is 100% or more.

5. The wound care arrangement according to any of the preceding claims, **characterised in that** the static friction coefficient of the covering unit (ASTM 1894-08) is between 0.8 and 1.5.

6. The wound care arrangement according to any of the preceding claims, **characterised in that** the sliding friction coefficient (ASTM 1894-08) of the covering unit is between 0.7 and 1.2.

7. The wound care arrangement according to any of the preceding claims, **characterised in that** the covering unit comprises polyurethane polymer, in particular an aromatic polyurethane polymer.

8. The wound care arrangement according to any of the preceding claims, **characterised by** a filler material for filling the wound space placed between the wound base and the covering unit, preferably, on the side of the suction connection facing the filler material, a drainage layer being provided for introducing the exudate to be removed by aspiration from the filler material into at least one aspiration opening of the suction connection.

9. The wound care arrangement according to any of the preceding claims, **characterised in that** the covering unit is associated with an adhering fastening means for the adhesive attachment of the covering unit to the skin surrounding the wound.

10. The wound care arrangement according to Claim 9, **characterised in that** the fastening means comprises a separate adhesive film, in particular a polyurethane film, by means of which the wound covering can be attached after application to the wound with respect to the skin surrounding the wound.

11. The wound care arrangement according to any of the preceding claims, **characterised in that** for forming a truncated arrangement, one axial end of the film tube is closed, in particular airtight.

12. The wound care arrangement according to any of the preceding claims, **characterised in that** the covering unit is associated with a support arrangement, in particular a supporting film, which is detachable from the wound after application to it.

13. The wound care arrangement according to any of the preceding claims, **characterised in that** the suction connection has an aspiration opening facing the wound space during use and a connecting device preferably designed as a tube socket for producing a connection between the aspiration opening and an aspiration tube.

14. The wound care arrangement according to Claim 13, **characterised in that** the aspiration opening penetrates a flange-like contact area of the suction connection and the contact area has, preferably surrounding the aspiration opening, and preferably equipped with adhesive means, a fastening area, by means of which the suction connection can be glued to a boundary area of the covering unit facing away from the wound space.

15. The wound care arrangement according to any of the preceding claims, **characterised in that** the film tube is completely closed in the peripheral direction and, preferably after application to the wound, is cut open in order to obtain a perforation associated with the aspiration opening, the perforation preferably being surrounded by the fastening area of the suction connection during use.

## Revendications

1. Agencement de pansement de plaie comprenant un moyen de recouvrement susceptible d'être fixé au niveau de la peau entourant une plaie et servant à créer un espace clos entourant la plaie, ainsi qu'un raccord d'aspiration (10) permettant de produire une dépression dans l'espace entourant la plaie, ledit moyen de recouvrement présentant un film tubulaire (200) dans lequel peut être introduite une extrémité du corps humain, **caractérisé en ce que** le moyen de recouvrement est perméable à la vapeur au moins par sections et **en ce que** la perméabilité à la vapeur du moyen de recouvrement est, au moins par sections, supérieure à 300 g/m²/24 h.

2. Agencement de pansement de plaie selon la revendication 1, **caractérisé en ce que** la perméabilité à la vapeur du moyen de recouvrement est inférieure à 10 000 g/m²/24 h, notamment inférieure à 5 000 g/m²/24 h et tout préférablement inférieure à 3 000 g/m²/24 h ou moins.

3. Agencement de pansement de plaie selon l'une des revendications précédentes, **caractérisé en ce que** le moyen de recouvrement présente un film de recouvrement de préférence transparent dont l'épaisseur est de 0,5 à 200 µm, notamment de 1 à 100 µm.

4. Agencement de pansement de plaie selon l'une des revendications précédentes, **caractérisé en ce que** l'allongement à la rupture du moyen de recouvrement est de 100 % ou plus.

5. Agencement de pansement de plaie selon l'une des revendications précédentes, **caractérisé en ce que** le coefficient de frottement statique du moyen de recouvrement (ASTM 1894-08) est compris entre 0,8 et 1,5.

6. Agencement de pansement de plaie selon l'une des revendications précédentes, **caractérisé en ce que** le coefficient de frottement dynamique (ASTM 1894-08) du moyen de recouvrement est compris entre 0,7 et 1,2.

7. Agencement de pansement de plaie selon l'une des revendications précédentes, **caractérisé en ce que** le moyen de recouvrement présente un polymère de polyuréthane, notamment un polymère de polyuréthane aromatique.

8. Agencement de pansement de plaie selon l'une des revendications précédentes, **caractérisé par** un matériau de remplissage mis en place pour remplir l'espace entourant la plaie entre le lit de la plaie et le moyen de recouvrement, et comportant une couche de drainage destinée à guider l'exsudat à éliminer du matériau de remplissage par au moins une ouverture d'aspiration du raccord d'aspiration, ladite couche de drainage étant située de préférence sur le côté du raccord d'aspiration qui est tourné vers le matériau de remplissage.

9. Agencement de pansement de plaie selon l'une des revendications précédentes, **caractérisé en ce qu'**il est associé au moyen de recouvrement un moyen de fixation adhésif permettant la fixation adhésive du moyen de recouvrement sur la peau entourant la plaie.

10. Agencement de pansement de plaie selon la revendication 9, **caractérisé en ce que** le moyen de fixation présente un film adhésif séparé, notamment un film de polyuréthane, permettant de fixer le dispositif de recouvrement de plaie, après disposition au niveau de la plaie, par rapport à la peau entourant la plaie.

11. Agencement de pansement de plaie selon l'une des revendications précédentes, **caractérisé en ce que** le film tubulaire est fermé en une extrémité axiale, notamment de manière étanche à l'air, pour créer un dispositif tronconique.

12. Agencement de pansement de plaie selon l'une des revendications précédentes, **caractérisé en ce qu'**il est associé au moyen de recouvrement un dispositif de support, notamment un film de support, qui est amovible après avoir été disposé sur la plaie.

13. Agencement de pansement de plaie selon l'une des revendications précédentes, **caractérisé en ce que** le raccord d'aspiration présente une ouverture d'aspiration tournée, à l'usage, vers l'espace entourant la plaie, ainsi qu'un dispositif de montage réalisé de préférence sous la forme d'un manchon tubulaire permettant de réaliser un montage entre l'ouverture d'aspiration et un tuyau d'aspiration.

14. Agencement de pansement de plaie selon la revendication 13, **caractérisé en ce que** l'ouverture d'aspiration traverse une zone de contact de type à collerette du raccord d'aspiration et **en ce que** la zone de contact présente une zone de fixation de préférence entourant l'ouverture d'aspiration et de préférence pourvue d'un adhésif et qui permet de coller le raccord d'aspiration sur une surface de délimitation du moyen de recouvrement détournée de l'espace entourant la plaie.

15. Agencement de pansement de plaie selon l'une des revendications précédentes, **caractérisé en ce que** le film tubulaire est totalement fermé dans le sens circonférentiel et est découpé, de préférence après avoir été disposé sur la plaie, pour obtenir un évidement associé à l'ouverture d'aspiration, ledit évidement étant, à l'usage, de préférence entouré par la zone de fixation du raccord d'aspiration.
